# EUROPEAN PATENT APPLICATION

(11) **EP 4 837 639 A2**
(43) Date of publication of application: **11.11.2026**
(21) Application number: 26192610.9
(22) Date of filing: 11.03.2021
(51) Int. Cl.: A61P 37/00

(54) **METHOD FOR TREATING INFLAMMATORY BOWEL DISEASE I**

(30) Priority: 11.03.2020 AU 2020900742
(62) Document number(s) of the earlier application(s) in accordance with Art. 76 EPC: 21712449.4 / 4 117 691
(71) Applicant: Mesoblast International Sàrl, 1217 Meyrin (CH)
(72) Inventor: ITESCU, Silviu, Melbourne, 30000 (AU)
(74) Representative: Thomann, William John

(57) **Abstract**

The present disclosure relates to method of treating or preventing inflammatory bowel disease (IBD) in a subject in need thereof, the method comprising administering to the subject a composition comprising mesenchymal lineage precursor or stem cells (MLPSCs).

## Description

### FIELD OF THE DISCLOSURE

The present disclosure relates to methods for treating or preventing inflammatory bowel disease (IBD) in a subject in need thereof.

### BACKGROUND

Inflammatory bowel disease (IBD) is a debilitating, relapsing condition that first emerges in young adulthood and can affect a patient throughout their life. IBD covers a group of disorders in which the gastrointestinal tract becomes inflamed. Major types of IBD include Crohn's disease, in which inflammation affects the full thickness of the bowel wall anywhere along the gastrointestinal tract, and ulcerative colitis, in which inflammation affects the inner lining (mucosa) of the colon and rectum.

Crohn's disease affects nearly two million people in the United States and millions more world-wide, and continues to increase in incidence for unknown reasons. Monoclonal antibodies have become the cornerstone of medical therapy for moderate to severe disease since the FDA approval of infliximab in 2006. However, their utility is limited by primary and secondary non-response and the risk of serious opportunistic infections. Furthermore, biologics can take a long time to demonstrate clinical improvement. Therefore, non-responding patients may be largely untreated during this period, becoming increasingly malnourished, anemic, and suffering from complications of their disease while awaiting evaluation of medical responsiveness.

Accordingly, there remains an unmet therapeutic need in patients with IBD and/or its associated conditions or symptoms with new treatment options being required.

### SUMMARY OF THE DISCLOSURE

The present inventors have surprisingly identified that early disease remission (day 28) can be achieved in subjects with inflammatory bowel disease by injecting mesenchymal precursor lineage or stem cells. Accordingly, in a first example, the present disclosure relates to a method of treating or preventing inflammatory bowel disease in a human subject in need thereof, the method comprising administering to the subject a composition comprising mesenchymal lineage precursor or stem cells (MLPSCs), wherein the composition is administered to gastrointestinal tract wall of the subject.

In an example, the composition is administered to the submucosal layer of the subjects gastrointestinal tract wall. In another example, the composition may be administered to a site of inflammation in the subjects gastrointestinal tract wall. In an example, the composition may be administered to the colon and/or rectum of the subject.

In an example, the composition is administered via intra-luminal injection. For example, the composition may be administered via endoscope. In another example, the method further comprises simultaneous or sequential intravenous administration of mesenchymal precursor lineage or stem cells to the subject.

In an example, the subject is refractory to at least one biologic therapy. In an example, the subject is only refractory to one biologic therapy. In an example, the subject is refractory to at least one anti-TNF therapy. In another example, the subject is refractory to steroid immunosuppressant and/or a biologic therapy.

In an example, the inflammatory bowel disease is Crohn's disease or ulcerative colitis. For example, the inflammatory bowel disease may be Crohn's disease. In an example, the Crohn's disease presents in the rectum and/or colon of the subject. In an example, the Crohn's disease is fistulizing Crohn's disease. In another example, the Crohn's disease is moderate to severe.

In an example, the subject has a partial clinical and/or endoscopic response at least 28 days after treatment. In another example, the subject has a partial clinical and/or endoscopic response at least 28 to 56 days after treatment. In an example, the partial clinical response is characterized by one or more or all of:
- >25% reduction in C-reactive protein (CRP);
- Decrease in CD Activity Index (CDAI) by <100 points;
- radiographic healing as assessed via MR enterography with improvement of inflammation.

In an example, the partial endoscopic response is characterized by one or both of:
- Decreased Simple Endoscopic Score for Crohn Disease (SES-CD) by >25% and an SES-CD < 50%;
- SES-CD score of 10-15.

In an example, the subject has a clinical and/or endoscopic response at least 28 days after treatment. In another example, the subject has a clinical and/or endoscopic response at least 28 to 56 days after treatment. In an example, a clinical response is characterized by one or more or all of:
- Reduction in CRP by >50%;
- Normalization of CRP;
- >100 point drop in CDAI;
- radiographic healing as assessed via MR enterography with improvement of inflammation.

In an example, an endoscopic response is characterized by one or both of:
- Decreased SES-CD by >25% but < 50%;
- SES-CD score of 5-10.

In an example, the subject is in clinical and/or endoscopic remission at least 28 days after treatment. In another example, the subject is in clinical and/or endoscopic remission at least 28 to 56 days after treatment. In an example, clinical remission is characterized by one or both of:
- normalization of CRP to <2.87 mg per litre;
- radiographic healing as assessed via MR enterography with improvement of inflammation.

In an example, endoscopic remission is characterized by one or both of:
- absence of mucosal ulceration;
- SES-CD score of 0-5.

In an example, MLPSCs are administered into the submucosal layer of the subjects colon wall. In another example, MLPSCs are administered to multiple sites in the subjects gastrointestinal tract wall.

In an example, the MLPSCs are mesenchymal stem cells (MSCs). In an example, the MLPSCs are allogeneic. For example, the MLPSCs may be allogeneic MSCs.

In an example, the subject has a CDAI greater than 300.

In an example, the MLPSCs are administered via an endoscope.

In an example, methods of the disclosure comprise administering between 1 x 10⁷ and 2 x 10⁸ cells. In another example, methods of the disclosure comprise administering between 1 x 10⁷ and 2 x 10⁸ cells to the gastrointestinal tract wall of the subject at two, three, four, five, six or more sites. For example, the MLPSCs may be administered at two, three, four, five, six or more sites in the subjects colon and/or rectum.

In an example, MLPSC compositions of the disclosure further comprise Plasma-Lyte A, dimethyl sulfoxide (DMSO), human serum albumin (HSA). For example, such compositions may comprise Plasma-Lyte A (70%), DMSO (10%), HSA (25%) solution, the HSA solution comprising 5% HSA and 15% buffer. In another examples, such compositions may comprise greater than 6.68x10⁶ viable cells/mL.

### BRIEF DESCRIPTION OF ACCOMPANYING FIGURES

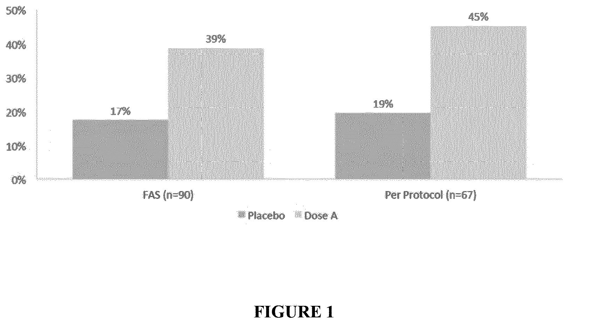

**FIGURE 1****:** Percentage of patients achieving CDAI score 150 or less at day 28. FAS: all randomized, at least one treatment, at least one post-baseline assessment; PP: all FAS without major protocol events.

### DETAILED DESCRIPTION

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter.

Those skilled in the art will appreciate that the disclosure described herein is susceptible to variations and modifications other than those specifically described. It is to be understood that the disclosure includes all such variations and modifications. The disclosure also includes all of the steps, features, compositions and compounds referred to or indicated in this specification, individually or collectively, and any and all combinations or any two or more of said steps or features.

The present disclosure is not to be limited in scope by the specific embodiments described herein, which are intended for the purpose of exemplification only. Functionally-equivalent products, compositions and methods are clearly within the scope of the disclosure, as described herein.

Any example disclosed herein shall be taken to apply *mutatis mutandis* to any other example unless specifically stated otherwise.

Unless specifically defined otherwise, all technical and scientific terms used herein shall be taken to have the same meaning as commonly understood by one of ordinary skill in the art (e.g., in cell culture, molecular genetics, stem cell differentiation, immunology, immunohistochemistry, protein chemistry, and biochemistry).

Unless otherwise indicated, the surgical techniques utilized in the present disclosure are standard procedures, well known to those skilled in the art.

Methods of obtaining and enriching a population of mesenchymal lineage stem or precursor cells are known in the art. For example, enriched populations of mesenchymal lineage stem or precursor cells can be obtained by the use of flow cytometry and cell sorting procedures based on the use of cell surface markers that are expressed on mesenchymal lineage stem or precursor cells.

All documents cited or referenced herein, and all documents cited or referenced in herein cited documents, together with any manufacturer's instructions, descriptions, product specifications, and product sheets for any products mentioned herein or in any document incorporated by reference herein, are hereby incorporated herein by reference in their entirety.

### Selected Definitions

The term "and/or", e.g., "X and/or Y" shall be understood to mean either "X and Y" or "X or Y" and shall be taken to provide explicit support for both meanings or for either meaning.

As used herein, the term about, unless stated to the contrary, refers to +/- 10%, more preferably +/- 5%, of the designated value.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

As used herein, the singular form "a", "an" and "the" include singular and plural references unless the context indicates otherwise.

By "isolated" or "purified" it is meant a cell which has been separated from at least some components of its natural environment. This term includes gross physical separation of the cells from its natural environment (e.g. removal from a donor). The term "isolated" includes alteration of the cell's relationship with the neighboring cells with which it is in direct by, for example, dissociation. The term "isolated" does not refer to a cell which is in a tissue section. When used to refer to the population of cells, the term "isolated" includes populations of cells which result from proliferation of the isolated cells of the disclosure.

The terms "passage", "passaging" or "sub-culture" are used in the context of the present disclosure to refer to known cell culture techniques that are used to keep cells alive and growing under cultured conditions for extended periods of time so that cell numbers can continually increase. The degree of sub-culturing a cell line has undergone is often expressed as "passage number," which is generally used to refer to the number of times cells have been sub-cultured. In an example, one passage comprises removing non-adherent cells and leaving adherent mesenchymal lineage precursor or stem cells. Such mesenchymal lineage precursor or stem cells can then be dissociated from the substrate or flask (e.g., by using a protease such as trypsin or collagenase), media can be added, optional washing (e.g., by centrifugation) may be performed, and then the mesenchymal lineage precursor or stem cells can be re-plated or reseeded to one or more culture vessels containing a greater surface area in total. The mesenchymal lineage precursor or stem cells can then continue to expand in culture. In another example, methods of removing non-adherent cells include steps of non-enzymatic treatment (e.g., with EDTA). In an example, mesenchymal lineage precursor or stem cells are passaged at or near confluence (e.g., about 75% to about 95% confluence). In an example, the mesenchymal lineage precursor or stem cells are seeded at a concentration of about 10%, about 15%, or about 20% cells/ml of culture medium.

The term "medium" or "media" as used in the context of the present disclosure, includes the components of the environment surrounding cells in culture. It is envisaged that the media contributes to and/or provides the conditions suitable to allow cells to grow. Media may be solid, liquid, gaseous or a mixture of phases and materials. Media can include liquid growth media as well as liquid media that do not sustain cell growth. Exemplary gaseous media include the gaseous phase that cells growing on a petri dish or other solid or semisolid support are exposed to.

The terms "gastrointestinal tract" or "GI tract" encompass the human organ system which spans from the mouth to the anus. The gastrointestinal tract encompasses mouth, esophagus, stomach and intestines. Accordingly, reference to a wall of the gastrointestinal tract in the present disclosure a wall of the encompasses mouth, esophagus, stomach and intestines. For the avoidance of doubt, the term "intestines" includes colon and rectum.

As used herein, the terms "treating", "treat" or "treatment" include administering a population of mesenchymal lineage stem or precursor cells and/or progeny thereof and/or soluble factors derived therefrom to thereby reduce or eliminate at least one symptom of inflammatory bowel disease. In an example, treatment includes administering a population of culture expanded mesenchymal lineage stem or precursor cells. In an example, the treatment induces partial clinical and/or endoscopic response. In an example, the partial clinical and/or endoscopic response is induced at least 25 days after treatment. In an example, the partial clinical and/or endoscopic response is induced at least 28 days after treatment. In an example, the partial clinical and/or endoscopic response is induced at least 30 days after treatment. In an example, the partial clinical and/or endoscopic response is induced at least 35 days after treatment. In another example, the partial clinical and/or endoscopic response is induced at least 28 to 65 days after treatment. In another example, the partial clinical and/or endoscopic response is induced at least 28 to 56 days after treatment.

In an example, a partial clinical response is characterized by one or more or all of:
- >25% reduction in C-reactive protein (CRP);
- Decrease in CD Activity Index (CDAI) by <100 points;
- radiographic healing as assessed via MR enterography with improvement of inflammation;
- Reduction in the Mayo Clinic score less than 3 points and decrease of less than 30% from baseline with a decrease of at least 2 points on the rectal bleeding sub-scale to an absolute rectal bleeding score of 1 or 2.

In an example, a partial endoscopic response is characterized by one or both of:
- Decreased Simple Endoscopic Score for Crohn Disease (SES-CD) by >25% and an SES-CD < 50%;
- SES-CD score of 10-15;
- No improvement in Mayo Clinic scale endoscopic sub-score that stays the same or decreases.

In an example, the treatment induces clinical and/or endoscopic response. In an example, the clinical and/or endoscopic response is induced at least 25 days after treatment. In an example, the clinical and/or endoscopic response is induced at least 28 days after treatment. In an example, the clinical and/or endoscopic response is induced at least 30 days after treatment. In an example, the clinical and/or endoscopic response is induced at least 35 days after treatment. In another example, the clinical and/or endoscopic response is induced at least 28 to 65 days after treatment. In another example, the clinical and/or endoscopic response is induced at least 28 to 56 days after treatment.

In an example, a clinical response is characterized by one or more or all of:
- Reduction in CRP by >50%;
- Normalization of CRP;
- ≥100 point drop in CDAI;
- radiographic healing as assessed via MR enterography with improvement of inflammation;
- Reduction in Mayo Clinic score by 3 and decrease of at least 30% from baseline with a decrease of at least 2 points on the rectal bleeding subscale to absolute rectal bleeding score of 1 or 2.

In an example, an endoscopic response is characterized by one or both of:
- Decreased SES-CD by >25% but < 50%;
- SES-CD score of 5-10;
- Mayo Clinic scale endoscopic sub-score decrease by at least one point.

In an example, the treatment induces clinical and/or endoscopic remission. In an example, the clinical and/or endoscopic remission is induced at least 25 days after treatment. In an example, the clinical and/or endoscopic remission is induced at least 28 days after treatment. In an example, the clinical and/or endoscopic remission is induced at least 30 days after treatment. In an example, the clinical and/or endoscopic remission is induced at least 35 days after treatment. In another example, the clinical and/or endoscopic remission is induced at least 28 to 65 days after treatment. In another example, the clinical and/or endoscopic remission is induced at least 28 to 56 days after treatment.

In an example, clinical remission is characterized by one or more or all of:
- normalization of CRP to <2.87 mg per litre;
- radiographic healing as assessed via MR enterography with improvement of inflammation.

In an example, endoscopic remission is characterized by one or both of:
- absence of mucosal ulceration;
- SES-CD score of 0-5.

In an example, treatment can induce reduced C-reactive protein (CRP); decreased CD Activity Index (CDAI); radiographic healing as assessed via MR enterography; decreased Simple Endoscopic Score for Crohn Disease (SES-CD), compared to the baseline value (i.e. control or before administration of mesenchymal lineage stem or precursor cells and/or progeny thereof and/or soluble factors derived therefrom).

The term "prevent" or "preventing" as used herein include administering a population of mesenchymal lineage stem or precursor cells and/or progeny thereof and/or soluble factors derived therefrom to thereby stop or inhibit the development of at least one symptom of inflammatory bowel disease.

The term "inflammatory bowel disease" (IBD) is used in the context of the present disclosure refer to inflammatory diseases of the gastrointestinal tract such as ulcerative colitis (UC), irritable bowel syndrome, irritable colon syndrome, Crohn's colitis and Crohn's disease (CD).

The term "ulcerative colitis (UC)" can include mild-to-moderate ulcerative colitis. Mild-to-moderate ulcerative colitis can be characterized by one or ,more or all of the following:
- A score of 4-10 on the ulcerative colitis-disease activity index (UC-DAI);
- A sigmoidoscopy score of > 4;
- A Physician's Global Assessment (PGA) score of > 2).

"Crohn's disease activity index (CDAI score)" is a research tool developed by WR Best and colleagues from the Midwest Regional Health Center in Illinois, in 1976 to quantify the symptoms of patients with Crohn's disease. The index is the most widely used instrument for evaluation of Crohn's disease activity (Sandborn et al. (2002) Gastroenterology., 122:512-530) and consists of eight factors/variables. The eight variables are summed after adjustment with a weighting factor. The components of the CDAI and weighting factors are shown in the following table:

| **Clinical or laboratory variable** | **Weighting factor** |
|---|---|
| Number of liquid or soft stools (sum of each day for seven days) | x2 |
| Abdominal pain (graded from 0 - 3 on severity) (sum of each day for seven days) | x5 |
| General well being, subjectively assessed from 0 (well) to 4 (terrible) (sum of each day for seven days) | x7 |
| Presence of Crohn's disease complications | x20 |
| Use of diphenoxylate or loperamide for diarrhea during the past week (0 = no; 1 = yes) | x30 |
| Presence of an abdominal mass (0 as none, 2 as questionable, 5 as definite) | x10 |
| Absolute deviation of Hematocrit from 47% in men and 42% in women | x6 |
| Percentage deviation from standard weight | x1 |

Total CDAI scores range from 0 to approximately 600 where the higher the score, the more active the disease. In an example, a CDAI score of less than 150 points denotes "clinical remission" of the Crohn's disease. In an example, between 150 to 219 points denotes "active mild Crohn's disease". In an example, between 220 to 450 points denotes "active moderate Crohn's disease". In an example, more than 450 points denotes "active severe Crohn's disease".

In an example, treatment induces ≥50 point drop in CDAI. In another example, treatment induces ≥75 point drop in CDAI. In another example, treatment induces ≥90 point drop in CDAI. In another example, treatment induces ≥100 point drop in CDAI. In another example, treatment induces ≥150 point drop in CDAI. In another example, treatment induces a 50 to 150 point drop in CDAI. In another example, treatment induces a 75 to 125 point drop in CDAI. In another example, treatment induces a 90 to 110 point drop in CDAI.

"C-reactive protein" or "CRP" is an inflammatory mediator whose levels are raised under conditions of acute inflammatory recurrence and rapidly normalize once the inflammation subsides. In an example, treatment reduces CRP by >20% from baseline. In another example, treatment reduces CRP by >30% from baseline. In another example, treatment reduces CRP by >40% from baseline. In another example, treatment reduces CRP by >50% from baseline. In another example, treatment reduces CRP by >60% from baseline. In another example, treatment reduces CRP by 20% to 60% from baseline. In another example, treatment reduces CRP by 30% to 50% from baseline. In another example, treatment reduces CRP to less than 2.95 mg per litre. In another example, treatment reduces CRP to less than 2.87 mg per litre. In another example, treatment normalizes CRP levels in the subject.

In an example, treatment provides a SES-CD score of 0-5. In another example, treatment provides a SES-CD score of 5-10. In another example, treatment provides a SES-CD score of 10-15. In another example, treatment provides a SES-CD score of 0-15.

In an example, methods of the present disclosure inhibit disease progression or disease complication in a subject. "Inhibition" of disease progression or disease complication in a subject means preventing or reducing the disease progression and/or disease complication in the subject.

The term "subject" as used herein refers to a human subject. For example, the subject can be an adult. In another example, the subject can be a child. In another example, the subject can be an adolescent. Terms such as "subject", "patient" or "individual" are terms that can, in context, be used interchangeably in the present disclosure.

Subjects treated according to the present disclosure may have symptoms indicative of an inflammatory bowel disease. For example, a subject may have gastrointestinal symptoms indicative of inflammatory bowel disease. Exemplary gastrointestinal symptoms include diarrhoea, constipation, nausea, vomiting, flatulence, cramping, bloating, abdominal pain, steatorrhea, rectal bleeding. In an example, a subject treated according to the present disclosure may present with one or more symptoms selected from the group consisting of fatigue, weakness and lethargy, iron deficiency, anaemia, vitamin and mineral deficiency, failure to thrive, delayed puberty, weight loss, bone and joint pain, recurrent mouth ulcers and/or swelling of mouth or tongue, altered mental alertness and irritability, skin rashes such as dermatitis, herpetiformis, easy bruising of the skin and regular reflux. In an example, the subject has previously failed at least one anti-TNF therapy. In an example, the subject has a contra-indication to biologic therapy.

In another example, the subject is 18-75 years of age. In another example, the subject has Crohn's disease. In another example, the subject has ulcerative colitis. In another example, the subject has Crohn's disease and ulcerative colitis. In another example, the subjects Crohn's disease presents in the intestine of the subject. In another example, the subjects Crohn's disease presents in the rectum and/or colon of the subject.

In another example, the subject has had Crohn's disease for at least 6 months duration. In another example, the subjects Crohn's disease is moderate to severe. In another example, the subjects Crohn's disease is fistulizing Crohn's disease (see for e.g. Gecse et al. 2013 United European Gastroenterol J., 1:206-213).

In another example, the subject has a CDAI greater than 200. In another example, the subject has a CDAI greater than 250. In another example, the subject has a CDAI greater than 300. In another example, the subject has a CDAI between 200 and 450. In another example, the subject has a CDAI between 250 and 400. In another example, the subject has a CDAI between 300 and 400.

In another example, the methods of the present disclosure prevent or treat subjects with active mild Crohn's disease. In another example, the methods of the present disclosure prevent or treat subjects with active moderate Crohn's disease. In another example, the methods of the present disclosure prevent or treat subjects with active severe Crohn's disease. In another example, the methods of the present disclosure prevent or treat subjects with active moderate or severe Crohn's disease. For example, the methods of the present disclosure may be used to prevent or treat subjects with active moderate Crohn's disease that are refractory to immunosuppressant and/or a biologic therapy. For example, a subjects Crohn's may be refractory to a TNF-alpha antagonist and/or a steroid.

As used herein the term "refractory" is used in the context of the present disclosure to refer to subjects that fail or are resistant to a certain treatment, such as "biologic therapy", e.g., treatment with a biologic such as infliximab or adalimumab. In an example, a subject is refractory to a biologic therapy if the next step in medical management is an escalation in medical management. In an example, a subject is refractory to a biologic therapy if the next step in medical management is an alternative biologic therapy. In another example, a subject is refractory to a biologic therapy if the next step in medical management is subtotal colectomy. In an example, the subject is refractory to a single biologic therapy. In this example, the subject has not been treated with more than one biologic therapy.

The term "biologic therapy" is used in the context of the present disclosure to refer to recombinant proteins that are derived or synthesized from living biological organisms. In an example, the biologic therapy is used for the treatment of an inflammatory conditions such as inflammatory bowel disease, such as Crohn's colitis. In an example, the biologic therapy is an antibody. For example, the biologic therapy can be a monoclonal antibody. In another example, the biologic therapy is an anti-TNF therapy. Examples of biologic therapies encompassed by the present disclosure include infliximab, adalimumab, certolizumab pegol, vedolizumab or ustekinumab. Accordingly, in an example, subjects encompassed by the present disclosure may be refractory to infliximab, adalimumab, certolizumab pegol, vedolizumab or ustekinumab.

As used herein, the term "genetically unmodified" refers to cells that have not been modified by transfection with a nucleic acid. For the avoidance of doubt, in the context of the present disclosure a mesenchymal lineage precursor or stem cell transfected with a nucleic acid encoding Ang1 would be considered genetically modified.

### Mesenchymal lineage precursor cells

As used herein, the term "mesenchymal lineage precursor or stem cell (MLPSC)" refers to undifferentiated multipotent cells that have the capacity to self-renew while maintaining multipotency and the capacity to differentiate into a number of cell types either of mesenchymal origin, for example, osteoblasts, chondrocytes, adipocytes, stromal cells, fibroblasts and tendons, or non-mesodermal origin, for example, hepatocytes, neural cells and epithelial cells. For the avoidance of doubt, a "mesenchymal lineage precursor cell" refers to a cell which can differentiate into a mesenchymal cell such as bone, cartilage, muscle and fat cells, and fibrous connective tissue.

The term "mesenchymal lineage precursor or stem cells" includes both parent cells and their undifferentiated progeny. The term also includes mesenchymal precursor cells, multipotent stromal cells, mesenchymal stem cells (MSCs), perivascular mesenchymal precursor cells, and their undifferentiated progeny.

Mesenchymal lineage precursor or stem cells can be autologous, allogeneic, xenogenic, syngenic or isogenic. Autologous cells are isolated from the same individual to which they will be reimplanted. Allogeneic cells are isolated from a donor of the same species. Xenogenic cells are isolated from a donor of another species. Syngenic or isogenic cells are isolated from genetically identical organisms, such as twins, clones, or highly inbred research animal models.

In an example, the mesenchymal lineage precursor or stem cells are allogeneic. In an example, the allogeneic mesenchymal lineage precursor or stem cells are culture expanded and cryopreserved.

Mesenchymal lineage precursor or stem cells reside primarily in the bone marrow, but have also shown to be present in diverse host tissues including, for example, cord blood and umbilical cord, adult peripheral blood, adipose tissue, trabecular bone and dental pulp. They are also found in skin, spleen, pancreas, brain, kidney, liver, heart, retina, brain, hair follicles, intestine, lung, lymph node, thymus, ligament, tendon, skeletal muscle, dermis, and periosteum; and are capable of differentiating into germ lines such as mesoderm and/or endoderm and/or ectoderm. Thus, mesenchymal lineage precursor or stem cells are capable of differentiating into a large number of cell types including, but not limited to, adipose, osseous, cartilaginous, elastic, muscular, and fibrous connective tissues. The specific lineage-commitment and differentiation pathway which these cells enter depends upon various influences from mechanical influences and/or endogenous bioactive factors, such as growth factors, cytokines, and/or local microenvironmental conditions established by host tissues.

The terms "enriched", "enrichment" or variations thereof are used herein to describe a population of cells in which the proportion of one particular cell type or the proportion of a number of particular cell types is increased when compared with an untreated population of the cells (e.g., cells in their native environment). In one example, a population enriched for mesenchymal lineage precursor or stem cells comprises at least about 0.1% or 0.5% or 1% or 2% or 5% or 10% or 15% or 20% or 25% or 30% or 50% or 75% mesenchymal lineage precursor or stem cells. In this regard, the term "population of cells enriched for mesenchymal lineage precursor or stem cells" will be taken to provide explicit support for the term "population of cells comprising X% mesenchymal lineage precursor or stem cells", wherein X% is a percentage as recited herein. The mesenchymal lineage precursor or stem cells can, in some examples, form clonogenic colonies, e.g. CFU-F (fibroblasts) or a subset thereof (e.g., 50% or 60% or 70% or 70% or 90% or 95%) can have this activity.

In an example of the present disclosure, the mesenchymal lineage precursor or stem cells are mesenchymal stem cells (MSCs). The MSCs may be a homogeneous composition or may be a mixed cell population enriched in MSCs. Homogeneous MSC compositions may be obtained by culturing adherent marrow or periosteal cells, and the MSCs may be identified by specific cell surface markers which are identified with unique monoclonal antibodies. A method for obtaining a cell population enriched in MSCs is described, for example, in U.S. Patent No. 5,486,359. Alternative sources for MSCs include, but are not limited to, blood, skin, cord blood, muscle, fat, bone, and perichondrium. In an example, the MSCs are allogeneic. In an example, the MSCs are cryopreserved. In an example, the MSCs are culture expanded and cryopreserved.

In another example, the mesenchymal lineage precursor or stem cells are CD29+, CD54+, CD73+, CD90+, CD102+, CD105+, CD106+, CD166+, MHC1+MSCs.

Isolated or enriched mesenchymal lineage precursor or stem cells can be expanded *in vitro* by culture. Isolated or enriched mesenchymal lineage precursor or stem cells can be cryopreserved, thawed and subsequently expanded *in vitro* by culture.

In one example, isolated or enriched mesenchymal lineage precursor or stem cells are seeded at 50,000 viable cells/cm² in culture medium (serum free or serum-supplemented), for example, alpha minimum essential media (αMEM) supplemented with 5% fetal bovine serum (FBS) and glutamine, and allowed to adhere to the culture vessel overnight at 37°C, 20% O₂. The culture medium is subsequently replaced and/or altered as required and the cells cultured for a further 68 to 72 hours at 37°C, 5% O₂.

As will be appreciated by those of skill in the art, cultured mesenchymal lineage precursor or stem cells are phenotypically different to cells *in vivo.* For example, in one embodiment they express one or more of the following markers, CD44, NG2, DC146 and CD140b. Cultured mesenchymal lineage precursor or stem cells are also biologically different to cells *in vivo,* having a higher rate of proliferation compared to the largely non-cycling (quiescent) cells *in vivo.*

In one example, the population of cells is enriched from a cell preparation comprising STRO-1+ cells in a selectable form. In this regard, the term "selectable form" will be understood to mean that the cells express a marker (e.g., a cell surface marker) permitting selection of the STRO-1+ cells. The marker can be STRO-1, but need not be. For example, as described and/or exemplified herein, cells (e.g., mesenchymal precursor cells) expressing STRO-2 and/or STRO-3 (TNAP) and/or STRO-4 and/or VCAM-1 and/or CD146 and/or 3G5 also express STRO-1 (and can be STRO-1bright). Accordingly, an indication that cells are STRO-1+ does not mean that the cells are selected solely by STRO-1 expression. In one example, the cells are selected based on at least STRO-3 expression, e.g., they are STRO-3+ (TNAP+).

Reference to selection of a cell or population thereof does not necessarily require selection from a specific tissue source. As described herein STRO-1+ cells can be selected from or isolated from or enriched from a large variety of sources. That said, in some examples, these terms provide support for selection from any tissue comprising STRO-1+ cells (e.g., mesenchymal precursor cells) or vascularized tissue or tissue comprising pericytes (e.g., STRO-1+ pericytes) or any one or more of the tissues recited herein.

In one example, the cells used in the present disclosure express one or more markers individually or collectively selected from the group consisting of TNAP+, VCAM-1+, THY-1+, STRO-2+, STRO-4+ (HSP-90β), CD45+, CD146+, 3G5+ or any combination thereof.

By "individually" is meant that the disclosure encompasses the recited markers or groups of markers separately, and that, notwithstanding that individual markers or groups of markers may not be separately listed herein the accompanying claims may define such marker or groups of markers separately and divisibly from each other.

By "collectively" is meant that the disclosure encompasses any number or combination of the recited markers or groups of markers, and that, notwithstanding that such numbers or combinations of markers or groups of markers may not be specifically listed herein the accompanying claims may define such combinations or subcombinations separately and divisibly from any other combination of markers or groups of markers.

As used herein the term "TNAP" is intended to encompass all isoforms of tissue non-specific alkaline phosphatase. For example, the term encompasses the liver isoform (LAP), the bone isoform (BAP) and the kidney isoform (KAP). In one example, the TNAP is BAP. In one example, TNAP as used herein refers to a molecule which can bind the STRO-3 antibody produced by the hybridoma cell line deposited with ATCC on 19 December 2005 under the provisions of the Budapest Treaty under deposit accession number PTA-7282.

Furthermore, in one example, the STRO-1+ cells are capable of giving rise to clonogenic CFU-F.

In one example, a significant proportion of the STRO-1+ cells are capable of differentiation into at least two different germ lines. Non-limiting examples of the lineages to which the STRO-1+ cells may be committed include bone precursor cells; hepatocyte progenitors, which are multipotent for bile duct epithelial cells and hepatocytes; neural restricted cells, which can generate glial cell precursors that progress to oligodendrocytes and astrocytes; neuronal precursors that progress to neurons; precursors for cardiac muscle and cardiomyocytes, glucose-responsive insulin secreting pancreatic beta cell lines. Other lineages include, but are not limited to, odontoblasts, dentin-producing cells and chondrocytes, and precursor cells of the following: retinal pigment epithelial cells, fibroblasts, skin cells such as keratinocytes, dendritic cells, hair follicle cells, renal duct epithelial cells, smooth and skeletal muscle cells, testicular progenitors, vascular endothelial cells, tendon, ligament, cartilage, adipocyte, fibroblast, marrow stroma, cardiac muscle, smooth muscle, skeletal muscle, pericyte, vascular, epithelial, glial, neuronal, astrocyte and oligodendrocyte cells.

In an example, mesenchymal lineage precursor or stem cells are obtained from a single donor, or multiple donors where the donor samples or mesenchymal lineage precursor or stem cells are subsequently pooled and then culture expanded.

Mesenchymal lineage precursor or stem cells encompassed by the present disclosure may also be cryopreserved prior to administration to a subject. In an example, mesenchymal lineage precursor or stem cells are culture expanded and cryopreserved prior to administration to a subject.

In an example, the present disclosure encompasses mesenchymal lineage precursor or stem cells as well as progeny thereof, soluble factors derived therefrom, and/or extracellular vesicles isolated therefrom. In another example, the present disclosure encompasses mesenchymal lineage precursor or stem cells as well as extracellular vesicles isolated therefrom. For example, it is possible to culture expand mesenchymal precursor lineage or stem cells of the disclosure for a period of time and under conditions suitable for secretion of extracellular vesicles into the cell culture medium. Secreted extracellular vesicles can subsequently be obtained from the culture medium for use in therapy.

The term "extracellular vesicles" as used herein, refers to lipid particles naturally released from cells and ranging in size from about 30 nm to as a large as 10 microns, although typically they are less than 200 nm in size. They can contain proteins, nucleic acids, lipids, metabolites, or organelles from the releasing cells (e.g., mesenchymal stem cells; STRO-1⁺ cells).

The term "exosomes" as used herein, refers to a type of extracellular vesicle generally ranging in size from about 30 nm to about 150 nm and originating in the endosomal compartment of mammalian cells from which they are trafficked to the cell membrane and released. They may contain nucleic acids (*e.g.,* RNA; microRNAs), proteins, lipids, and metabolites and function in intercellular communication by being secreted from one cell and taken up by other cells to deliver their cargo.

### Culture expansion of the cells

In an example, mesenchymal lineage precursor or stem cells are culture expanded. "Culture expanded" mesenchymal lineage precursor or stem cells media are distinguished from freshly isolated cells in that they have been cultured in cell culture medium and passaged (i.e. sub-cultured). In an example, culture expanded mesenchymal lineage precursor or stem cells are culture expanded for about 4 - 10 passages. In an example, mesenchymal lineage precursor or stem cells are culture expanded for at least 5, at least 6, at least 7, at least 8, at least 9, at least 10 passages. For example, mesenchymal lineage precursor or stem cells can be culture expanded for at least 5 passages. In an example, mesenchymal lineage precursor or stem cells can be culture expanded for at least 5 - 10 passages. In an example, mesenchymal lineage precursor or stem cells can be culture expanded for at least 5 - 8 passages. In an example, mesenchymal lineage precursor or stem cells can be culture expanded for at least 5 - 7 passages. In an example, mesenchymal lineage precursor or stem cells can be culture expanded for more than 10 passages. In another example, mesenchymal lineage precursor or stem cells can be culture expanded for more than 7 passages. In these examples, stem cells may be culture expanded before being cryopreserved to provide an intermediate cryopreserved MLPSC population. In an example, compositions of the disclosure are prepared from an intermediate cryopreserved MLPSC population. For example, an intermediate cryopreserved MLPSC population can be further culture expanded prior to administration as is discussed further below. Accordingly, in an example, mesenchymal lineage precursor or stem cells are culture expanded and cryopreserved. In an embodiment of these examples, mesenchymal lineage precursor or stem cells can be obtained from a single donor, or multiple donors where the donor samples or mesenchymal lineage precursor or stem cells are subsequently pooled and then culture expanded. In an example, the culture expansion process comprises:
- i. expanding by passage expansion the number of viable cells to provide a preparation of at least about 1 billion of the viable cells, wherein the passage expansion comprises establishing a primary culture of isolated mesenchymal lineage precursor or stem cells and then serially establishing a first non-primary (P1) culture of isolated mesenchymal lineage precursor or stem cells from the previous culture;
- ii. expanding by passage expansion the P1 culture of isolated mesenchymal lineage precursor or stem cells to a second non-primary (P2) culture of mesenchymal lineage precursor or stem cells; and,
- iii. preparing and cryopreserving an in-process intermediate mesenchymal lineage precursor or stem cells preparation obtained from the P2 culture of mesenchymal lineage precursor or stem cells; and,
- iv. thawing the cryopreserved in-process intermediate mesenchymal lineage precursor or stem cells preparation and expanding by passage expansion the in-process intermediate mesenchymal lineage precursor or stem cells preparation.

In an example, the expanded mesenchymal lineage precursor or stem cell preparation has an antigen profile and an activity profile comprising:
- i. less than about 0.75% CD45+ cells;
- ii. at least about 95% CD105+ cells;
- iii. at least about 95% CD166+ cells.

In an example, the expanded mesenchymal lineage precursor or stem cell preparation is capable of inhibiting IL2Ra expression by CD3/CD28-activated PBMCs by at least about 30% relative to a control.

In an example, culture expanded mesenchymal lineage precursor or stem cells are culture expanded for about 4 - 10 passages, wherein the mesenchymal lineage precursor or stem cells have been cryopreserved after at least 2 or 3 passages before being further culture expanded. In an example, mesenchymal lineage precursor or stem cells are culture expanded for at least 1, at least 2, at least 3, at least 4, at least 5 passages, cryopreserved and then further culture expanded for at least 1, at least 2, at least 3, at least 4, at least 5 passages before being administered or further cryopreserved.

In an example, the majority of mesenchymal lineage precursor or stem cells in compositions of the disclosure are of about the same generation number (i.e., they are within about 1 or about 2 or about 3 or about 4 cell doublings of each other). In an example, the average number of cell doublings in the present compositions is about 20 to about 25 doublings. In an example, the average number of cell doublings in the present compositions is about 9 to about 13 (e.g., about 11 or about 11.2) doublings arising from the primary culture, plus about 1, about 2, about 3, or about 4 doublings per passage (for example, about 2.5 doublings per passage). Exemplary average cell doublings in present compositions are any of about 13.5, about 16, about 18.5, about 21, about 23.5, about 26, about 28.5, about 31, about 33.5, and about 36 when produced by about 1, about 2, about 3, about 4, about 5, about 6, about 7, about 8, about 9, and about 10 passages, respectively.

The process of mesenchymal lineage precursor or stem cell isolation and *ex vivo* expansion can be performed using any equipment and cell handing methods known in the art. Various culture expansion embodiments of the present disclosure employ steps that require manipulation of cells, for example, steps of seeding, feeding, dissociating an adherent culture, or washing. Any step of manipulating cells has the potential to insult the cells. Although mesenchymal lineage precursor or stem cells can generally withstand a certain amount of insult during preparation, cells are preferably manipulated by handling procedures and/or equipment that adequately performs the given step(s) while minimizing insult to the cells.

In an example, mesenchymal lineage precursor or stem cells are washed in an apparatus that includes a cell source bag, a wash solution bag, a recirculation wash bag, a spinning membrane filter having inlet and outlet ports, a filtrate bag, a mixing zone, an end product bag for the washed cells, and appropriate tubing, for example, as described in US 6,251,295, which is hereby incorporated by reference.

In an example, a mesenchymal lineage precursor or stem cell composition according to the present disclosure is 95% homogeneous with respect to being CD105 positive and CD166 positive and being CD45 negative. In an example, this homogeneity persists through *ex vivo* expansion; i.e. though multiple population doublings. In an example, the composition comprises at least one therapeutic dose of mesenchymal lineage precursor or stem cells and the mesenchymal lineage precursor or stem cells comprise less than about 1.25% CD45+ cells, at least about 95% CD105+ cells, and at least about 95% CD166+ cells. In an example, this homogeneity persists after cryogenic storage and thawing, where the cells also generally have a viability of about 70% or more.

In an example, compositions of the disclosure comprise mesenchymal lineage precursor or stem cells which express substantial levels of TNFR1, for example greater than 13 pg of TNFR1 per million mesenchymal lineage precursor or stem cells. In an example, this phenotype is stable throughout *ex vivo* expansion and cryogenic storage. In an example, expression of levels of TNFR1 in the range of about 13 to about 179 pg (e.g. about 13 pg to about 44 pg) per million mesenchymal lineage precursor or stem cells is associated with a desirous therapeutic potential which also persists through *ex vivo* expansion and cryopreservation.

In an example, the culture expanded mesenchymal lineage precursor or stem cells express Tumor necrosis factor receptor 1 (TNFR1) in an amount of at least 110 pg/ml. For example, the mesenchymal lineage precursor or stem cells can express TNFR1 in an amount of at least 150 pg/ml, or at least 200 pg/ ml, or at least 250 pg/ml, or at least 300 pg/ml, or at least 320 pg/ml, or at least 330 pg/ml, or at least 340 pg/ml, or at least 350 pg/ml.

In an example, the mesenchymal lineage precursor or stem cells express TNFR1 in an amount of at least 13 pg/10⁶ cells. For example, the mesenchymal lineage precursor or stem cells express TNFR1 in an amount of at least 15 pg/10⁶ cells, or at least 20 pg/10⁶ cells, or at least 25 pg/10⁶ cells, or at least 30 pg/10⁶ cells, or at least 35 pg/10⁶ cells, or at least 40 pg/10⁶ cells, or at least 45 pg/10⁶ cells, or at least 50 pg/10⁶ cells.

In another example, mesenchymal lineage precursor or stem cells disclosed herein inhibit IL-2Rα expression on T-cells. In an example, mesenchymal lineage precursor or stem cells can inhibit IL-2Rα expression by at least about 30%, alternatively at least about 35%, alternatively at least about 40%, alternatively at least about 45%, alternatively at least about 50%, alternatively at least about 55%, alternatively at least about 60.

In an example, compositions of the disclosure comprise at least one therapeutic dose of mesenchymal lineage precursor or stem cells which, for example, can comprise at least about 100 million cells or about 125 million cells.

### Modification of the cells

The mesenchymal lineage precursor or stem cells of the present disclosure may be altered in such a way that upon administration, lysis of the cell is inhibited. Alteration of an antigen can induce immunological non-responsiveness or tolerance, thereby preventing the induction of the effector phases of an immune response (e.g., cytotoxic T cell generation, antibody production etc.) which are ultimately responsible for rejection of foreign cells in a normal immune response. Antigens that can be altered to achieve this goal include, for example, MHC class I antigens, MHC class II antigens, LFA-3 and ICAM-1.

The mesenchymal lineage precursor or stem cells may also be genetically modified to express proteins of importance for the differentiation and/or maintenance of striated skeletal muscle cells. Exemplary proteins include growth factors (TGF-β, insulin-like growth factor 1 (IGF-1), FGF), myogenic factors (e.g. myoD, myogenin, myogenic factor 5 (Myf5), myogenic regulatory factor (MRF)), transcription factors (e.g. GATA-4), cytokines (e.g. cardiotropin-1), members of the neuregulin family (e.g. neuregulin 1, 2 and 3) and homeobox genes (e.g. Csx, tinman and NKx family).

### Compositions of the disclosure

In one example of the present disclosure the mesenchymal lineage precursor or stem cells and/or progeny thereof and/or soluble factor derived therefrom are administered in the form of a composition. In one example, such a composition comprises a pharmaceutically acceptable carrier and/or excipient. Accordingly, in an example, compositions of the disclosure can comprise culture expanded mesenchymal lineage precursor or stem cells.

The terms "carrier" and "excipient" refer to compositions of matter that are conventionally used in the art to facilitate the storage, administration, and/or the biological activity of an active compound (see, e.g., Remington's Pharmaceutical Sciences, 16th Ed., Mac Publishing Company (1980). A carrier may also reduce any undesirable side effects of the active compound. A suitable carrier is, for example, stable, e.g., incapable of reacting with other ingredients in the carrier. In one example, the carrier does not produce significant local or systemic adverse effect in recipients at the dosages and concentrations employed for treatment.

Suitable carriers for the present disclosure include those conventionally used, e.g., water, saline, aqueous dextrose, lactose, Ringer's solution, a buffered solution, hyaluronan and glycols are exemplary liquid carriers, particularly (when isotonic) for solutions. Suitable pharmaceutical carriers and excipients include starch, cellulose, glucose, lactose, sucrose, gelatin, malt, rice, flour, chalk, silica gel, magnesium stearate, sodium stearate, glycerol monostearate, sodium chloride, glycerol, propylene glycol, water, ethanol, and the like.

In another example, a carrier is a media composition, e.g., in which a cell is grown or suspended. For example, such a media composition does not induce any adverse effects in a subject to whom it is administered.

Exemplary carriers and excipients do not adversely affect the viability of a cell and/or the ability of a cell to reduce, prevent or delay metabolic syndrome and/or obesity.

In one example, the carrier or excipient provides a buffering activity to maintain the cells and/or soluble factors at a suitable pH to thereby exert a biological activity, e.g., the carrier or excipient is phosphate buffered saline (PBS). PBS represents an attractive carrier or excipient because it interacts with cells and factors minimally and permits rapid release of the cells and factors, in such a case, the composition of the disclosure may be produced as a liquid for direct application to the blood stream or into a tissue or a region surrounding or adjacent to a tissue, e.g., by injection.

The mesenchymal lineage precursor or stem cells and/or progeny thereof and/or soluble factor derived therefrom can also be incorporated or embedded within scaffolds that are recipient-compatible and which degrade into products that are not harmful to the recipient. These scaffolds provide support and protection for cells that are to be transplanted into the recipient subjects. Natural and/or synthetic biodegradable scaffolds are examples of such scaffolds.

A variety of different scaffolds may be used successfully in the practice of the disclosure. Exemplary scaffolds include, but are not limited to biological, degradable scaffolds. Natural biodegradable scaffolds include collagen, fibronectin, and laminin scaffolds. Suitable synthetic material for a cell transplantation scaffold should be able to support extensive cell growth and cell function. Such scaffolds may also be resorbable. Suitable scaffolds include polyglycolic acid scaffolds, (e.g., as described by Vacanti, et al. J. Ped. Surg. 23:3-9 1988; Cima, et al. Biotechnol. Bioeng. 38:145 1991; Vacanti, et al. Plast. Reconstr. Surg. 88:753-9 1991); or synthetic polymers such as polyanhydrides, polyorthoesters, and polylactic acid.

In another example, the mesenchymal lineage precursor or stem cells and/or progeny thereof and/or soluble factor derived therefrom may be administered in a gel scaffold (such as Gelfoam from Upjohn Company).

The compositions described herein may be administered alone or as admixtures with other cells. The cells of different types may be admixed with a composition of the disclosure immediately or shortly prior to administration, or they may be co-cultured together for a period of time prior to administration.

In one example, the composition comprises an effective amount or a therapeutically or prophylactically effective amount of mesenchymal lineage precursor or stem cells and/or progeny thereof and/or soluble factor derived therefrom. For example, the composition comprises about 1x10⁵ stem cells to about 1x10⁹ stem cells or about 1.25x10³ stem cells to about 1.25x10⁷ stem cells/kg (80 kg subject). The exact amount of cells to be administered is dependent upon a variety of factors, including the age, weight, and sex of the subject, and the extent and severity of the disorder being treated.

In an example, 50 x 10⁶ to 200 x 10⁷ cells are administered. In other examples, 60 x 10⁶ to 200 x 10⁶ cells or 75 x 10⁶ to 150 x 10⁶ cells are administered. In an example, 75 x 10⁶ cells are administered. In another example, 150 x 10⁶ cells are administered.

In an example, , the composition comprises greater than 5.00x10⁶ viable cells/mL. In another example, the composition comprises greater than 5.50x10⁶ viable cells/mL. In another example, the composition comprises greater than 6.00x10⁶ viable cells/mL. In another example, the composition comprises greater than 6.50x10⁶ viable cells/mL. In another example, the composition comprises greater than 6.68x10⁶ viable cells/mL.

In an example, the mesenchymal lineage precursor or stem cells comprise at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99% of the cell population of the composition.

Compositions of the disclosure may be cryopreserved. Cryopreservation of mesenchymal lineage precursor or stem cells can be carried out using slow-rate cooling methods or 'fast' freezing protocols known in the art. Preferably, the method of cryopreservation maintains similar phenotypes, cell surface markers and growth rates of cryopreserved cells in comparison with unfrozen cells.

The cryopreserved composition may comprise a cryopreservation solution. The pH of the cryopreservation solution is typically 6.5 to 8, preferably 7.4.

The cryopreservation solution may comprise a sterile, non-pyrogenic isotonic solution such as, for example, PlasmaLyte A^{™}. 100 mL of PlasmaLyte A^{™} contains 526 mg of sodium chloride, USP (NaCl); 502 mg of sodium gluconate (C₆H₁₁NaO₇); 368 mg of sodium acetate trihydrate, USP (C₂H₃NaO₂•3H₂O); 37 mg of potassium chloride, USP (KCl); and 30 mg of magnesium chloride, USP (MgCl₂•6H₂O). It contains no antimicrobial agents. The pH is adjusted with sodium hydroxide. The pH is 7.4 (6.5 to 8.0).

The cryopreservation solution may comprise Profreeze^{™}. The cryopreservation solution may additionally or alternatively comprise culture medium, for example, αMEM.

To facilitate freezing, a cryoprotectant such as, for example, dimethylsulfoxide (DMSO), is usually added to the cryopreservation solution. Ideally, the cryoprotectant should be nontoxic for cells and patients, nonantigenic, chemically inert, provide high survival rate after thawing and allow transplantation without washing. However, the most commonly used cryoprotector, DMSO, shows some cytotoxicity . Hydroxylethyl starch (HES) may be used as a substitute or in combination with DMSO to reduce cytotoxicity of the cryopreservation solution.

The cryopreservation solution may comprise one or more of DMSO, hydroxyethyl starch, human serum components and other protein bulking agents. In one example, the cryopreserved solution comprises about 5% human serum albumin (HSA) and about 10% DMSO. The cryopreservation solution may further comprise one or more of methycellulose, polyvinyl pyrrolidone (PVP) and trehalose.

In one embodiment, cells are suspended in 42.5% Profreeze^{™}/50% αMEM/7.5% DMSO and cooled in a controlled-rate freezer.

The cryopreserved composition may be thawed and administered directly to the subject or added to another solution, for example, comprising HA. Alternatively, the cryopreserved composition may be thawed and the mesenchymal lineage precursor or stem cells resuspended in an alternate carrier prior to administration.

In an example, cellular compositions of the disclosure can comprise Plasma-Lyte A, dimethyl sulfoxide (DMSO) and human serum albumin (HSA). For example, compositions of the disclosure may comprise Plasma-Lyte A (70%), DMSO (10%), HSA (25%) solution, the HSA solution comprising 5% HSA and 15% buffer.

In an example, the compositions described herein may be administered as a single dose.

In some examples, the compositions described herein may be administered over multiple doses. For example, at least 2, at least 3, at least 4, at least 5, at least 6, at least 7, at least 8, at least 9, at least 10 doses.

In one example, the mesenchymal lineage precursor or stem cells can be culture expanded prior to administration to a subject. Various methods of cell culture are known in the art. In an example, mesenchymal lineage precursor or stem cells are culture expanded for about 4 - 10 passages. In an example, mesenchymal lineage precursor or stem cells are culture expanded for at least 4, at least 5, at least 6, at least 7, at least 8, at least 9, at least 10 passages. In an example, mesenchymal lineage precursor or stem cells are culture expanded for at least 5 passages. In these examples, stem cells may be culture expanded before being cryopreserved.

In an example, mesenchymal lineage precursor or stem cells are culture expanded in a serum free medium prior to administration.

In some examples, the cells are contained within a chamber that does not permit the cells to exit into a subject's circulation but permits factors secreted by the cells to enter the circulation. In this manner soluble factors may be administered to a subject by permitting the cells to secrete the factors into the subject's circulation. Such a chamber may equally be implanted at a site in a subject to increase local levels of the soluble factors, e.g., implanted in or near a gastrointestinal wall.

In an example, mesenchymal lineage precursor or stem cells may be administered to a wall of a subjects gastrointestinal tract. In an example, mesenchymal lineage precursor or stem cells are administered topically to an intraluminal wall of the gastrointestinal tract. In another example, mesenchymal lineage precursor or stem cells may be administered locally. For example, mesenchymal lineage precursor or stem cells can be administered into a wall of a subjects gastrointestinal tract. For example, mesenchymal lineage precursor or stem cells can be administered into the submucosae of a wall of a subjects gastrointestinal tract. In an example, mesenchymal lineage precursor or stem cells can be administered to a site of inflammation in a subjects gastrointestinal tract wall. For example, mesenchymal lineage precursor or stem cells can be administered into a site of inflammation in a subjects gastrointestinal tract wall. In these examples, the site of inflammation may be endoscopicaly confirmed prior to administration. For example, endoscopic confirmation can be based on visual inspection by a trained physician and/or histological analysis of endoscopic biopsy. In an example, the wall of the gastrointestinal tract is an intestinal wall. For example, mesenchymal lineage precursor or stem cells can be administered to a subjects colon wall and/or bowel wall. In an example, mesenchymal lineage precursor or stem cells can be administered directly into the submucosae of a subjects colon wall and/or bowel wall. In an example, mesenchymal lineage precursor or stem cells can be administered directly into the submucosae of a subjects colon wall and/or rectal wall. In another example, compositions of the disclosure can be administered via intra-luminal injection.

In various examples, a dose of cells may need to be administered to multiple sites in a subjects gastrointestinal tract. The number of sites of administration required per dose may be dictated by the number of cells being administered. For example, a dose of around 75 million cells may need to be administered to five sites in the gastrointestinal tract. In another example, a dose of around 150 million cells may need to be administered to 15 sites in the gastrointestinal tract. In other examples, a dose may need to be administered to a subjects gastrointestinal tract at two, three, four, five, six, seven, 8, 9, 10, 11, 12, 13, 14, 15, 16, 17, 18, 19, 20 or more sites. In an embodiment of these examples, doses are administered to a wall of a subjects cecum, proximal transverse colon, distal transverse colon descending colon, sigmoid colon, and rectum. In this embodiment, a dose can be administered to a wall of a subjects cecum, proximal transverse colon, distal transverse colon descending colon, sigmoid colon, and rectum at one, two, three, four, five or more sites.

In an example, mesenchymal lineage precursor or stem cells are administered via endoscope. For example, mesenchymal lineage precursor or stem cells can be injected into the submucosae of a subjects gastrointestinal tract wall via endoscope. In an example, the endoscope is used to visually identify a site of inflammation before mesenchymal lineage precursor or stem cells are administered directly into the site of inflammation.

It will be appreciated by persons skilled in the art that numerous variations and/or modifications may be made to the above-described embodiments, without departing from the broad general scope of the present disclosure. The present embodiments are, therefore, to be considered in all respects as illustrative and not restrictive.

The following specific examples are to be construed as merely illustrative, and not limitative of the remainder of the disclosure in any way whatsoever. Without further elaboration, it is believed that one skilled in the art can, based on the description herein, utilize the present invention to its fullest extent.

The present application claims priority from Australian Provisional Patent Application 2020900742 filed 11 March 2020, the entire contents of which are incorporated herein by reference.

### EXAMPLES

### Ex-vivo culture-expanded adult allogeneic bone marrow derived mesenchymal stem cells (MSCs), for the treatment of medically refractory Crohn's colitis

### Composition

The composition is comprised of culture-expanded mesenchymal stromal cells (ceMSC) isolated from the bone marrow of healthy adult donors. The final composition comprises ceMSC formulated in Plasma-Lyte A (70%), dimethyl sulfoxide (DMSO, 10%) and human serum albumin (HSA) (25%) solution (20%, comprising 5% HSA and 15% buffer) at a concentration of ≥6.68x106 viable cells/mL. Each dose vial contains 3.8 mL of cryopreserved cell suspension (total cells per vial ≥25x10⁶).

### Objectives

### Primary objectives

To determine the safety of endoscopic delivery of MSCs, ex vivo expanded allogeneic bone marrow derived MSCs, for the treatment of medically refractory Crohn's colitis.

### Secondary objectives

To assess in preliminary fashion the response of luminal healing induced by the endoscopic delivery of MSCs, ex vivo expanded allogeneic bone marrow derived MSCs, for the treatment of medicaly refractory Crohn's colitis.

Clinically
- Decreased number of 24 hour bowel movements ;
- Decreased blood in the stool;
- Decreased C-reactive protein serum levels;
- Decreased Crohn's disease activity index (CDAI score).

Radiographically
- Cross sectional imaging with magnetic resonance (MR) enterography.

Endoscopic and Histopathology
- Improved Simple Endoscopic Score for Crohns' disease (SES-CD) on colonoscopy;
- Improved histologic healing on endoscopic biopsy or surgical pathology as compared to pre-MSC delivery endoscopic biopsies.

### Subjects

Twenty four patients will have their luminal disease treated with MSCs at a dose of 75 million (n=12; 8 treatment, 4 control) or 150 million (n=12; 8 treatment, 4 control). MSCs will be delivered via targeted endoscpic delivery into the submucosal layer of the colon wall in the operating room. The MSC dose escalation will be conducted over two doseage groups of patients who will be allocated to treatment control in a 2:1 fashion four times (8:4 ratio in each doseage group). Twelve patients will receive 75 million cells and twelve will receive 150 million cells.

### Inclusion Criteria:

- Males and Females 18-75 years of age;
- Crohn's colitis of at least 6 months duration with medically refractory symptoms who has failed one anti-TNF therapy, with a next step of subtotal colectomy or escalation in medical management;
- Exposure to corticosteroids, 5-ASA drugs, thiopurines, methotrexate, anti-TNF therapy, anti-integrin and anti-interleukin are permitted but a washout period of 2 weeks for corticosteroids, 5-ASA, thiopurines, methotrexate will be performed, and a 4 week washout period of any biologic therapy;
- No colonic dysplasia and malignancy as ruled out by colonoscopy within 30 days of MSC delivery;
- Must have failed at least one anti-TNF or have a contra-indication to biologic therapy.

### Primary endpoint

The primary endpoint of this study is to determine the safety and feasibility of endoscopic injection of MSC, for treatment of Crohn's colitis.

### Secondary endpoints

### Clinical and endoscopic remission:

- Clinical Healing:
   ∘ Normalization of CRP to <2.87 mg per liter;
   ∘ CDAI drops to <150.
- Radiographic Healing:
   ∘ MR enterography with improvement of inflammation.
- Endoscopic healing:
   ∘ Absence of mucosal ulceration and SES-CD score of 0-5.

### Clinical and endoscopic response:

- Clinical Healing:
   ∘ Reduction in CRP by >50% or normalization;
   ∘ >100 point drop in CDAI.
- Radiographic Healing:
   ∘ MR enterography with improvement of inflammation.
- Endoscopic healing:
   ∘ Decreased SES-CD by >50% or to a score of 5-10.

### Partial clinical and endoscopic response:

- Clinical Healing:
   ∘ >25% reduction of CRP;
   ∘ decrease in CDAI by <100 points.
- Radiographic Healing:
   ∘ MR enterography with improvement in inflammation.
- Endoscopic healing:
   ∘ Decreased SES-CD by >25% but < 50% or to score of 10-15.

### Lack of Response:

- Clinical Healing:
   ∘ No improvement.
- Radiographic Healing:
   ∘ MR enterography without resolution of inflammation.
- Endoscopic healing:
   ∘ No improvement in SES-CD.

### Treatment regimen

Subjects receive either 75 million cells or 150 million cells (25 million per 3.8 mL of Plasma-Lyte^{®} A supplemented with human serum albumin (5%) and dimethyl sulfoxide (10%)) after randomization to treatment with MSC versus control with normal saline. The first cohort receive a dose of 75 million MSCs or normal saline, the next cohort of twelve patients receive 150 million MSCs or normal saline. For the 75 million dosage, the 75 million cells are suspended in 11.4 mL which is delivered in the cecum, proximal transverse colon, distal transverse colon descending colon, sigmoid colon, rectum, at 1.9 mL in each location. For the 150 million dosage, 22.8 mL is delivered in each previously mentioned location as 3 injections (1.3 mL each) in the 12, 6, and 9 o'clock positions of the colon/rectal wall. During the procedure, an adult colonoscope will be used. A 23-gauge single use sclerotherapy needle will be used to deliver the cells into the submucosal layer as evident by a small bleb raised in the submucosa. At each injection site, the MSC injection will be followed by 0.5 mL of Plasma-Lyte A in order to flush the sclerotherapy needle of any remaining MSCs.)

### Visit 1 (Screening/Baseline) MSC treated subjects

Patients will have the following tests and procedures completed at this visit:
- Eligibility (inclusion/exclusion checklist) at the time of a gastroenterologic or surgical consultation for change in biologic therapy or subtotal colectomy for medically refractory Crohn's colitis;
- Written informed consent;
- A washout period for the following medications:
   ∘ 2 weeks for 5-ASA, corticosteroids, immunomodulator therapy including azathioprine, methotrexate, and 6-mercaptopurine;
   ∘ 4 weeks for biologics: anti TNF, anti integrin, and interleukin.
- Medical & surgical history;
- General exam, including abdominal exam and vital signs (BP, pulse rate, respiratory rate and temperature);
- Crohn's Disease activity Index (CDAI) score;
- Inflammatory Bowel Disease Questionnaire (IBDQ) score will be obtained;
- Magnetic resonance enterography (MRE) if not previously performed in the last 30 days;
- Colonoscopy with biopsy, if not previously performed in the last 30 days;
- Rule out Cytomegalovirus colitis (CMV colitis);
- Simple endoscopic score for Crohn's disease (SES-CD);
- Laboratory studies including:
   ∘ A urine pregnancy test will be performed for women of child bearing potential (WOCBP) only
   ∘ Liver function tests, AST/ALT
   ∘ Acute Hepatitis Panel
   ∘ Human immunodeficiency virus (HIV)
   ∘ Complete blood count (CBC)
   ∘ Complete metabolic panel (CMP)
   ∘ Pre-albumin
   ∘ C-Reactive protein (CRP)
   ∘ Erthrocyte sedimentation rate (ESR)
   ∘ Clostridium difficile, fecal (C.diff)
   ∘ Calprotectin, Fecal
   ∘ Concomitant medications
- Adverse events (including change in medical management or operative management, and report any side effects to medications, and any postoperative complications)

### Visit 2 (Day 0 - Treatment)

Within 7 days prior to Visit 2, patients will have the following tests and procedures completed at this visit:
- General exam, including abdominal exam and vital signs;
- Inflammatory Bowel Disease Questionnaire (IBDQ) score;
- CDAI score;
- Randomizaton to treatment group or control group;
- Colonoscopy (will be used to administer MSCs);
- Concomitant medications;
- Adverse events;
- Delivery of MSCs or normal saline.

### Visit 3 (Day 1)

The following day the following tests and procedures will be completed:
- General exam, including abdominal exam and vital signs;
- Medical surgical history since last visit;
- CDAI score;
- Concomitant medications ;
- Adverse events.

### Visit 4 (Week 4 +/- 3 days)

The following tests and procedures completed at this visit:
- General exam, including abdominal exam and vital signs;
- Medical surgical history since last visit;
- Flexible sigmoidoscopy with biopsy;
- Inflammatory Bowel Disease Questionnaire (IBDQ) score;
- CDAI score;
- Concomitant medications;
- Adverse events.

### Visit 5 (Week 6 +/- 3 days)

The following tests and procedures completed at this visit:
- General exam, including abdominal exam and vital signs;
- Medical surgical history since last visit;
- Flexible sigmoidoscopy with biopsy;
- Inflammatory Bowel Disease Questionnaire (IBDQ) score;
- CDAI score;
- Concomitant medications;
- Adverse events.

### Visit 6 (3 months +/- 7 days)

The following tests and procedures completed at this visit:
- General exam, including abdominal exam and vital signs;
- Medical surgical history since last visit;
- Colonoscopy with SES-CD and biopsy;
- Inflammatory Bowel Disease Questionnaire (IBDQ) score;
- CDAI score;
- MRE;
- Laboratory workup:
   ∘ CBC;
   ∘ CMP;
   ∘ Pre Albumin;
   ∘ CRP;
   ∘ ESR;
   ∘ Calprotectin, fecal.
- Concomitant medications;
- Adverse events.

### Visit 7 (6 month; +/- 7 days)

The following tests and procedures completed at this visit:
- General exam, including abdominal exam and vital signs;
- Medical surgical history since last visit;
- Inflammatory Bowel Disease Questionnaire (IBDQ) score;
- CDAI score;
- MRE;
- Laboratory workup:
   ∘CBC;
   ∘ CMP;
   ∘ Pre Albumin;
   ∘ CRP;
   ∘ ESR;
   ∘ Calprotectin, fecal.
- Concomitant medications;
- Adverse events.

### Visit 8 (Month 9, +/- 14 days)

The following tests and procedures completed at this visit:
- General exam, including abdominal exam and vital signs;
- Medical surgical history since last visit;
- Inflammatory Bowel Disease Questionnaire (IBDQ) score;
- CDAI score;
- MRE;
- Laboratory workup:
   ∘ CBC;
   ∘ CMP;
   ∘ Pre Albumin;
   ∘CRP;
   ∘ ESR;
- Calprotectin, fecal.
- Concomitant medications;
- Adverse events.

### Visit 9 (Month 12, +/- 14 days)

The following tests and procedures completed at this visit:
- General exam, including abdominal exam and vital signs;
- Medical surgical history since last visit;
- Inflammatory Bowel Disease Questionnaire (IBDQ) score;
- CDAI score;
- MRE;
- Colonoscopy with biopsy (in treatment patients only).
- Laboratory workup:
   ∘CBC;
   ∘ CMP;
   ∘ Pre Albumin;
   ∘ CRP;
   ∘ ESR;
   ∘ Calprotectin, fecal.
- Concomitant medications;
- Adverse events.

### Visit 10 (15 months, +/- 14 days)

The following tests and procedures completed at this visit:
- General exam, including abdominal exam and vital signs;
- Medical surgical history since last visit;
- Inflammatory Bowel Disease Questionnaire (IBDQ) score;
- CDAI score;
- MRE;
- Colonoscopy with biopsy in the control arm (12 months from MSC treatment);
- Laboratory workup:
   ∘ CBC;
   ∘ CMP;
   ∘ Pre Albumin;
   ∘ CRP;
   ∘ ESR;
   ∘Calprotectin, fecal.
- Concomitant medications;
- Adverse events.

### Initial Results

Improved endoscopic healing and clinical healing was observed in a male patient with Crohn's colitis 6 weeks after administration of 75 million MSCs. 3 month follow up data had not yet been obtained from this patient. The patients baseline SES-CD was 16 and baseline CDAI was 294.

Improved endoscopic healing and clinical healing was observed in a female patient with Crohn's colitis 6 weeks after administration of 150 million MSCs. Endoscopic and clinical assessment three months after administration of therapy revealed that the patients disease was in remission. The patients baseline SES-CD was 22.

Improved clinical healing was observed in a male patient with ulcerative colitis 6 weeks after administration of 150 million MSCs. The patients baseline mayo score was 7. 6 weeks after administration of MSCs, the patients mayo score reduced to 3. 3 month follow up data had not yet been obtained from this patient.

### Prior Analysis

Sub-group analyses were conducted to explore the possible identification of a patient group which was most responsive to therapy, including single biologic refractory and multi-biologic refractory Crohn's disease, and fistulizing disease. These data are summarized in the Tables below and Figure 1.

There was a clear demonstration of early (Day 28) remission in patients with moderate to severe active Crohn's disease who had failed conventional therapy, steroids and a TNF-alpha inhibitor, with statistically significant response rates compared to controls (p=0.02; Figure 1). Evidence of sustained remission was demonstrated when comparing response rates from day 28 through day 56.

### Day 28 primary endpoint in population treated with one biologic.

| **One Biologic, Observed** | **Placebo** | | **Treatment Dose A** | | **-value comparison between Placebo and Treatment P** |
|---|---|---|---|---|---|
| FAS Proportion of Patients achieving CDAI score ≤150 at Day 28 | N=46 | 8/46 (17.4%) | N=44 | 17/44 (38.6%) | P=0.021 |
| PP Proportion of Patients achieving CDAI score ≤150 at Dav 28 | N=36 | 7/36 (19.4%) | N=31 | 14/31 (45.2%) | P=0.021 |
| Note: p values from large sample binomial test (two sided) | | | | | |

### Response rates from day 28 through day 56.

| **PP, One Biologic, Observed** | **Placebo (n=36)** | **Treatment Dose A (n=31) (*@* day 0, 3, 7, 14)** | **P-value comparison between Placebo and Treatment** |
|---|---|---|---|
| Proportion of Patients achieving CDAI score ≤150 at Days 28 and 56 | 2/30 (6.7%) | 11/28 (39.3%) | P=0.003 |
| Proportion of Patients achieving 100 point reduction in CDAI score at Days 28 and 56 | 14/30 (46.7%) | 16/28 (57.1%) | P=0.43 |
| Proportion of Patients achieving 70 point reduction in CDAI score at Days 28 and 56 | 16/30 (53.3%) | 22/28 (78.6%) | P=0.043 |

The invention provides, in particular, the following:
1. A method of treating or preventing inflammatory bowel disease in a human subject in need thereof, the method comprising administering to the subject a composition comprising mesenchymal lineage precursor or stem cells (MLPSCs), wherein the composition is administered to the gastrointestinal tract wall of the subject.
2. The method of item 1, wherein the composition is administered to the submucosal layer of the subjects gastrointestinal tract wall.
3. The method of item 1 or item 2, wherein the composition is administered to a site of inflammation in the subjects gastrointestinal tract wall.
4. The method according to any one of items 1 to 3, wherein the composition is administered to the colon and/or rectum of the subject.
5. The method according to any one of items 1 to 4, wherein the composition is administered via intra-luminal injection.
6. The method according to any one of items 1 to 5, wherein the subject is refractory to at least one anti-TNF therapy.
7. The method according to any one of items 1 to 6, wherein the subject is refractory to steroid immunosuppressant and/or a biologic therapy.
8. The method according to any one of items 1 to 7, wherein the inflammatory bowel disease is Crohn's disease or ulcerative colitis.
9. The method of item 8, wherein the inflammatory bowel disease is Crohn's disease.
10. The method of item 9, wherein the Crohn's disease presents in the rectum and/or colon of the subject.
11. The method according to any one of items 1 to 10, wherein the subject has a partial clinical and/or endoscopic response at least 28 days after treatment.
12. The method according to any one of items 1 to 10, wherein the subject has a partial clinical and/or endoscopic response at least 28 to 56 days after treatment.
13. The method of item 11 or 12 wherein partial clinical response is characterized by one or more or all of:
   - >25% reduction in C-reactive protein (CRP);
   - Decrease in CD Activity Index (CDAI) by <100 points;
   - radiographic healing as assessed via MR enterography with improvement of inflammation.
14. The method of item 11 or 12 wherein partial endoscopic response is characterized by one or both of:
   - Decreased Simple Endoscopic Score for Crohn Disease (SES-CD) by >25% and an SES-CD < 50%;
   - SES-CD score of 10-15.
15. The method according to any one of items 1 to 10, wherein the subject has a clinical and/or endoscopic response at least 28 days after treatment.
16. The method according to any one of items 1 to 10, wherein the subject has a clinical and/or endoscopic response at least 28 to 56 days after treatment.
17. The method of item 15 or 16 wherein clinical response is characterized by one or more or all of:
   - Reduction in CRP by >50%;
   - Normalization of CRP;
   - ≥100 point drop in CDAI;
   - radiographic healing as assessed via MR enterography with improvement of inflammation.
18. The method of item 15 or 16 wherein endoscopic response is characterized by one or both of:
   - Decreased SES-CD by >25% but < 50%;
   - SES-CD score of 5-10.
19. The method according to any one of items 1 to 10, wherein the subject is in clinical and/or endoscopic remission at least 28 days after treatment.
20. The method according to any one of items 1 to 10, wherein the subject is in clinical and/or endoscopic remission at least 28 to 56 days after treatment.
21. The method of item 19 or 20 wherein clinical remission is characterized by one or both of:
   - normalization of CRP to <2.87 mg per litre;
   - radiographic healing as assessed via MR enterography with improvement of inflammation.
22. The method of item 19 or 20 wherein endoscopic remission is characterized by one or both of:
   - absence of mucosal ulceration;
   - SES-CD score of 0-5.
23. The method according to any one of items 1 to 22, wherein the MLPSCs are administered into the submucosal layer of the subjects colon wall.
24. The method according to any one of items 1 to 23, wherein the MLPSCs are administered to multiple sites in the subjects gastrointestinal tract wall.
25. The method according to any one of items 1 to 24, wherein the MLPSCs are mesenchymal stem cells (MSCs).
26. The method according to any one of items 1 to 25, wherein the MLPSCs are allogeneic.
27. The method according to any one of items 8 to 26, wherein the Crohn's disease is moderate to severe.
28. The method according to any one of items 1 to 27, wherein the subject has a CDAI greater than 300.
29. The method according to any one of items 8 to 28, wherein the Crohn's disease is fistulizing Crohn's disease.
30. The method according to any one of items 1 to 29, wherein the mesenchymal lineage precursor or stem cells (MLPSCs) are administered via an endoscope.
31. The method according to any one of items 1 to 29 which comprises administering between 1 x 10⁷ and 2 x 10⁸ cells.
32. The method according to any one of items 1 to 29 which comprises administering between 1 x 10⁷ and 2 x 10⁸ cells to the gastrointestinal tract wall of the subject at two, three, four, five, six or more sites.
33. The method according to any one of items 1 to 31, wherein the composition further comprises Plasma-Lyte A, dimethyl sulfoxide (DMSO), human serum albumin (HSA).
34. The method according to any one of items 1 to 32, wherein the composition further comprises Plasma-Lyte A (70%), DMSO (10%), HSA (25%) solution, the HSA solution comprising 5% HSA and 15% buffer.
35. The method according to any one of items 1 to 33, wherein the composition comprises greater than 6.68x10⁶ viable cells/mL.

## Claims

1. A composition comprising mesenchymal lineage precursor or stem cells (MLPSCs) for use in a method of treating or preventing active moderate or severe Crohn's disease in a human subject in need thereof, wherein the composition is directly administered to the submucosal layer of the gastrointestinal tract wall of the subject.

2. The composition for use according to claim 1, wherein the composition is administered to a site of inflammation in the subjects gastrointestinal tract wall.

3. The method according to claim 1 or 2, wherein the composition is:
- administered to the colon and/or rectum of the subject;
- administered into the submucosal layer of the subject's colon wall;
- administered to multiple sites in the subjects gastrointestinal tract wall; and/or
- administered via an endoscope.

4. The composition for use according to any one of claims 1 to 3, wherein the subject is refractory to at least one anti-TNF therapy, or wherein the subject is refractory to steroid immunosuppressant and/or a biologic therapy.

5. The composition for use according to any one of claims 1 to 4, wherein the subject has a partial clinical and/or endoscopic response at least 28 days after treatment, the subject has a clinical and/or endoscopic response at least 28 days after treatment, or the subject is in clinical and/or endoscopic remission at least 28 days after treatment;
wherein partial clinical response is **characterized by** one or more or all of:
- >25% reduction in C-reactive protein (CRP);
- Decrease in CD Activity Index (CDAI) by <100 points;
- radiographic healing as assessed via MR enterography with improvement of inflammation, and
wherein partial endoscopic response is **characterized by** one or both of:
- Decreased Simple Endoscopic Score for Crohn Disease (SES-CD) by >25% and an SES-CD < 50%;
- SES-CD score of 10-15,
wherein clinical response is **characterized by** one or more or all of:
- Reduction in CRP by >50%;
- Normalization of CRP;
- >100 point drop in CDAI;
- radiographic healing as assessed via MR enterography with improvement of inflammation;
wherein endoscopic response is **characterized by** one or both of:
- Decreased SES-CD by >25% but < 50%;
- SES-CD score of 5-10;
wherein clinical remission is **characterized by** one or both of:
- normalization of CRP to <2.87 mg per litre;
- radiographic healing as assessed via MR enterography with improvement of inflammation; and
wherein endoscopic remission is **characterized by** one or both of:
- absence of mucosal ulceration;
- SES-CD score of 0-5.

6. The composition for use according to any one of claims 1 to 5, wherein the subject has a partial clinical and/or endoscopic response at least 28 to 56 days after treatment, the subject has a clinical and/or endoscopic response at least 28 to 56 days after treatment, or the subject is in clinical and/or endoscopic remission at least 28 to 56 days after treatment;
wherein partial clinical response is **characterized by** one or more or all of:
- >25% reduction in C-reactive protein (CRP);
- Decrease in CD Activity Index (CDAI) by <100 points;
- radiographic healing as assessed via MR enterography with improvement of inflammation, and
wherein partial endoscopic response is **characterized by** one or both of:
- Decreased Simple Endoscopic Score for Crohn Disease (SES-CD) by >25% and an SES-CD < 50%;
- SES-CD score of 10-15,
wherein clinical response is **characterized by** one or more or all of:
- Reduction in CRP by >50%;
- Normalization of CRP;
- >100 point drop in CDAI;
- radiographic healing as assessed via MR enterography with improvement of inflammation;
wherein endoscopic response is **characterized by** one or both of:
- Decreased SES-CD by >25% but < 50%;
- SES-CD score of 5-10,
wherein clinical remission is **characterized by** one or both of:
- normalization of CRP to <2.87 mg per litre;
- radiographic healing as assessed via MR enterography with improvement of inflammation; and
wherein endoscopic remission is **characterized by** one or both of:
- absence of mucosal ulceration;
- SES-CD score of 0-5.

7. The composition for use according to any one of claims 1 to 6, wherein the subject has a partial clinical response at least 28 days after treatment **characterized by** all of:
- >25% reduction in C-reactive protein (CRP);
- Decrease in CD Activity Index (CDAI) by <100 points;
- radiographic healing as assessed via MR enterography with improvement of inflammation.

8. The composition for use according to any one of claims 1 to 7, wherein the subject has a partial endoscopic response at least 28 days after treatment **characterized by** both of:
- Decreased Simple Endoscopic Score for Crohn Disease (SES-CD) by >25% and an SES-CD < 50%; and
- SES-CD score of 10-15.

9. The composition for use according to any one of claims 1 to 8, wherein the MLPSCs are mesenchymal stem cells (MSCs) and/or wherein the MLPSCs are allogeneic.

10. The composition for use according to any one of claims 1 to 9, wherein the subject has a CDAI greater than 300.

11. The composition for use according to any one of claims 1 to 10, which comprises a total dose of between 1 x 10⁷ and 2 x 10⁸ cells.

12. The composition for use according to any one of claims 1 to 11, which comprises a total dose of 7.5 x 10⁷ or 1.5 x 10⁸ cells.

13. The composition for use according to any one of claims 1 to 12, which comprises a total dose of between 1 x 10⁷ and 2 x 10⁸ cells that is directly administered to the submucosal layer of the gastrointestinal tract wall of the subject at two, three, four, five, six or more sites.

14. The composition for use according to any one of claims 1 to 13, wherein the composition further comprises Plasma-Lyte A (70%), dimethyl sulfoxide (DMSO) (10%), human serum albumin (HSA) (25%) solution, the HSA solution comprising 5% HSA and 15% buffer, optionally wherein the composition comprises greater than 6.68x10⁶ viable cells/mL.

15. The composition for use according to any one of claims 1 to 9 or 11 to 14, wherein the subject has a CDAI between 250 and 400.
